# EUROPÄISCHE PATENTSCHRIFT

(11) **EP 2 968 021 B1**
(45) Veröffentlichungstag und Bekanntmachung des Hinweises auf die Patenterteilung: **27.09.2017**
(21) Anmeldenummer: 14721743.4
(22) Anmeldetag: 13.03.2014
(51) Int. Cl.: A61F 13/10

(54) **FINGERLING**
FINGER-STALL
DOIGTIER

(30) Priorität: 14.03.2013 DE 102013102609
(43) Veröffentlichungstag der Anmeldung: 20.01.2016
(73) Patentinhaber: Technische Universität Dresden, 01069 Dresden (DE)
(72) Erfinder: SCHULTZ, Jurek, 01328 Dresden (DE); FITZE, Guido, 01309 Dresden (DE)
(74) Vertreter: Riechelmann & Carlsohn Patentanwälte
(86) Internationale Anmeldenummer: PCT/DE2014/100088
(87) Internationale Veröffentlichungsnummer: WO 2014/139518

(56) Entgegenhaltungen:
- DE-C- 375 555
- FR-A1- 2 715 832
- GB-A- 446 944
- GB-A- 191 319 869
- US-A- 835 803

## Beschreibung

Die Erfindung betrifft einen Fingerling, der insbesondere bei der Behandlung von Fingerendglieddefekten verwendet werden kann. Sie betrifft ferner ein Kit, das derartige Fingerlinge umfasst.

Das Fingerendglied bildet das distale Ende eines Fingers. Es umfasst die Fingerbeere, die auch als Fingerkuppe bezeichnet wird. Nach außen wird die Fingerkuppe durch Haut abgegrenzt, die über Bindegewebe am distalen Fingergliedknochen befestigt ist. Die Fingerkuppen werden als Teil des Greifapparates beim Greifen belastet. Bei Menschen sind die Fingerkuppen insbesondere am Spitz-, Schreib- und Schlüsselgriff beteiligt. Die Haut menschlicher Fingerkuppen weist eine Vielzahl unterschiedlicher Rezeptoren auf, beispielsweise Mechanorezeptoren, Schmerzrezeptoren und Wärmerezeptoren. Diese Rezeptoren sorgen für die hohe Sensibilität der Fingerkuppen, so dass die Fingerkuppen neben der Greiffunktion auch eine sensorische Funktion besitzen.

Die Behandlung von Verletzungen der Fingerkuppe soll sowohl deren Greiffunktion als auch deren sensorische Funktion so weit wie möglich wiederherstellen. Dabei sollen die Länge der Fingerkuppe, deren Sensibilität, Belastbarkeit und ästhetisches Erscheinungsbild erhalten oder wiederhergestellt sowie Überempfindlichkeiten vermieden werden. Während kleinere Verletzungen, d. h. Verletzungen der Haut und des Bindegewebes im Millimeterbereich, durch sekundäre Wundheilung, d. h. unter Narbenbildung, behoben werden können, können größere Verletzungen, d. h. Verletzungen, an denen der Fingerknochen beteiligt ist, darüber hinaus komplexere Behandlungsverfahren der plastischen Chirurgie erfordern. Dazu gehören insbesondere die Abdeckung mit Hauttransplantaten, lokalen Lappen oder neurovaskulär gestielten Lappenplastiken. Einzelheiten können Richter, M., Fingerkuppendefekte, Obere Extremität (5) 2010, 6-13, entnommen werden.

Operative Versorgungen sind jedoch kostenintensive Behandlungen durch hochspezialisiertes Personal und mit einer Narkose, Narbenbildung und regelmäßigem Verbandswechsel verbunden. Eine Restitutio ad integrum ist nicht möglich.

Es hat sich jedoch herausgestellt, dass die Behandlung von Fingerendglieddefekten mit Semiokklusivverbänden selbst dann vorteilhaft sein kann, wenn größere Verletzungen vorliegen. Ein solcher Semiokklusivverband kann mittels Gaze hergestellt werden. In beiden Fällen kann die Defektoberfläche mit Silbersulfadiazin behandelt werden. Bekannt sind ferner Semiokklusivverbände, bei denen eine spezielle Folie eingesetzt wird, die die Ausbildung eines spezifischen Wundmilieus an der Wunde ermöglichen soll. Es wird angenommen, dass dieses Wundmilieu, das eine Reihe von Wachstumsfaktoren umfasst, welche die Synthese von Kollagen fördern, eine schnelle Reepithelialisierung ermöglicht. In der Praxis werden diese Verbände häufig als Okklusivverbände bezeichnet, obwohl diese Verbände tatsächlich keine okklusiven, d. h. dichten Verbände sind. Alle bekannten Folien, Salben-Gazen, Gummihandschuhe etc., die zur Herstellung dieser Verbände nach dem Stand der Technik eingesetzt werden, sind mit Leckagen verbunden, so dass sie keine okklusiven, sondern semiokklusive Verbände sind. Die Bildung von Leckagen ist auf mehrere Faktoren zurückzuführen, insbesondere auf den Umstand, dass Finger schwitzen, Kapillarkräfte wirken und zumindest in den ersten 7 bis 14 Tagen nach Anlegen des Verbandes ständig neue Wundflüssigkeit gebildet wird.

Semiokklusivverbände sind insbesondere bei der Behandlung von Fingerendglieddefekten der Typen 1 bis 4 gemäß der Allen-Einteilung angezeigt, die sich jedoch nur auf transversale Verletzungen bezieht. Bei Defekten vom Typ 1 liegt keine Knochenbeteiligung vor, und das Nagelbett ist nur im geringen Umfang betroffen. Bei Defekten vom Typ 2 liegt eine Knochenbeteiligung vor, außerdem ist das Nagelbett, bezogen auf seine Länge, zu wenigstens 50 % erhalten. Auch bei nicht ganz transversalen, also leicht schrägen palmaren Verletzungen, die ansonsten Typ 2 gleichen, können Semiokklusivverbände angewendet werden. Bei ausgedehnten, stark schrägen palmaren Verletzungen sind bisher die Methoden der plastischen Chirurgie angeraten.

Die für die Semiokklusivverbände verwendeten Folien werden unter Abschluss des Defektes an dessen Rändern befestigt. Dabei soll ein Hohlraum zwischen der Defektoberfläche und der Folie ausgebildet werden, wobei der Hohlraum "dem Defekt entsprechen" soll. Die Folie wird ferner mittels Pflaster proximal von dem Defekt fixiert. Schließlich wird ein Verband um Folie und Pflaster gelegt. Richter, a. a. O., hält einen zumindest wöchentlichen Wechsel der Folie für erforderlich. In der Praxis waren Folienwechsel aller zwei bis drei Wochen oder erstmals nach vier Wochen notwendig. Die Dauer der Behandlungen beträgt zwischen vier und sechs Wochen. Einzelheiten hierzu sind Richter, a. a. O., zu entnehmen.

Der Einsatz der Folien zur Okklusion der Wunden ist jedoch mit einer Vielzahl von Nachteilen verbunden. Das Aufbringen der Folien ist umständlich. Es wird teilweise durch die Blutfeuchte des Fingers erschwert und ist gerade bei unkooperativen, kindlichen Patienten schwierig. Mittels der Folie lässt sich kein der natürlichen Form der Fingerkuppe nachempfundener Raum bilden. Die Folie bietet keinen mechanischen Schutz der Wunde vor Stößen etc. Ein solcher Schutz kann nur durch zusätzliche, die Hand unnötig immobilisierende Verbände und Schienungen bewerkstelligt werden. Auch die Ruhigstellung des betroffenen Fingers erfordert zusätzliche Schienen. Ein Verbandswechsel ist aufwendig, weil er das Entfernen der Schiene, der Umverbände und der Klebefolien, anschließend das Reinigen und Trocknen des Fingers zum kompletten Neuaufbringen des Verbandes erfordert. Auch eine atraumatische Asservierung von Wundsekret ohne direkten Kontakt zur Wundfläche ist nicht möglich. Außerdem besteht auch keine Möglichkeit zur Applikation von heilungsfördernden Mitteln in die Wunde, ohne die Okklusion zu entfernen und die Regeneration damit zu stören. Schließlich treten häufig übermäßige Undichtigkeiten des feuchten Raumes unter dem konventionellen Folienverband auf.

De Boer et al., JBJS (Br), 63-B (4) 1981, 545-547, beschreiben die Verwendung eines Fingerlings bei der Behandlung von Fingerendglieddefekten. Der Fingerling wurde von einem "gewöhnlichen", aus Gummi bestehenden Chirurgie-Handschuh abgeschnitten. Zunächst wurde die gesäuberte Wunde mit der Silbersulfadiazin-Creme bestrichen, dann wurde der Fingerling über den Finger gezogen und sein proximales Ende mittels eines Klebbandes am Finger befestigt. Allerdings ist der Fingerling ebenso undicht wie die Folienverbände, so dass Wundflüssigkeit aus dem Fingerling austrat, was eine gesonderte Belehrung der Patienten erforderte und zudem die Ausbildung des heilungsfördernden Wundmilieus beeinträchtigt. Ein weiterer Nachteil ist die ungenaue Ausbildung des Hohlraumes zwischen der Innenseite des Fingerlings und der Defektoberfläche. Schließlich ist der Wechsel des Fingerlings genauso umständlich wie der Wechsel der Folien. Der von de Boer et al. vorgeschlagene Fingerling hat sich daher in der Praxis nicht gegen die Folien durchsetzen können.

US 835,803 beschreibt einen Fingerling für Nadelgeschwüre. Der Fingerling weist einen Ballon auf, der über einen Hals mit einem Körper verbunden ist. Aus GB 446 944 und GB 19,869 sind Fingerlinge zum Schutz verletzter Finger bekannt.

Aus DE 375 555 ist eine Nervenschutzvorrichtung für Maschinenschreiber und Maschinensetzer bekannt. Die Nervenschutzvorrichtung sorgt für ein Luftpolster zwischen der Fingerspitze und dem äußeren Ende der Vorrichtung. GB 540,241 beschreibt eine Vorrichtung zum Auftragen einer Substanz auf ein Körpergewebe. Dazu ist eine Kammer in einem Fingerling ausgebildet. Die Substanz kann mittels des Fingers aus der Kammer herausgedrückt werden, ohne dass eine Verbindung zwischen dem Innenraum des Fingerlings und der Kammer besteht.

Aufgabe der Erfindung ist es, die Nachteile nach dem Stand der Technik zu beseitigen. Es soll insbesondere ein Fingerling angegeben werden, der eine einfache und bessere Behandlung von Fingerendglieddefekten erlaubt.

Diese Aufgabe wird durch die Merkmale der Ansprüche 1 und 9 gelöst. Zweckmäßige Ausgestaltungen der Erfindungen ergeben sich aus den Merkmalen der Unteransprüche.

Nach Maßgabe der Erfindung ist ein Fingerling vorgesehen, der insbesondere für die Behandlung von Fingerendglieddefekten geeignet ist und ein geschlossenes distales Ende, ein offenes proximales Ende und einen Innenraum aufweist, wobei in dem Fingerling an dessen distalem Ende eine Kammer ausgebildet ist und wobei die Kammer mit dem Innenraum des Fingerlings in Flüssigkeitskommunikation steht. Der Fingerling weist einen Verstärkungsbereich auf, der sich von dem proximalen Ende des Fingerlings zu den Wandungen der Kammer erstreckt.

Zur Anwendung wird der erfindungsgemäße Fingerling mit seinem offenen proximalen Ende über den Finger gezogen, der den Defekt aufweist. Das Fingerendglied gelangt dabei in einen Abschnitt des Innenraums, der an die Kammer angrenzt. Damit wird der Defekt von dem Mantel des Fingerlings und der Scheidewand zwischen Innenraum und Kammer umhüllt, so dass der Fingerendglieddefekt mittels des Fingerlings semiokklusiv eingeschossen wird. Die sich im Verlauf des Heilungsprozesses bildende Flüssigkeit kann in die Kammer eintreten. Damit bildet die Kammer ein Reservoir für die Wundflüssigkeit. Wundflüssigkeit, die in die Kammer eintritt und sich dort sammelt, kann aus der Kammer entnommen werden, beispielsweise mittels einer Kanüle, die von außen durch die Kammerwandung geführt wird. Der Hohlraum der Kammer ist vorzugsweise kugel- oder ellipsenförmig, wobei andere Formen möglich sind. Es können erfindungsgemäße Fingerlinge mit unterschiedlich großen Kammern vorgesehen sein, was eine Auswahl eines Fingerlings mit einer Kammer einer gewünschten Größe ermöglicht. Dabei kann ein Fingerling mit einer umso größeren Kammer gewählt werden, je größer der Fingerendglieddefekt ist.

Die Materialstärke und die Form der Kammer sind so gewählt, dass mit einer herkömmlichen Kanüle aus der Kammer jederzeit unkompliziert Wundflüssigkeit, vorzugsweise um 500 µl, für diagnostische und wissenschaftliche Zwecke entnommen werden kann, ohne den Fingerling zu entfernen, die Gewebsregeneration zu stören oder dem Patienten Schmerzen zu verursachen. Der Fingerling bleibt trotzdem weitestgehend dicht und läuft auch nach vielfacher Punktion nicht aus. Vorzugsweise liegt das Innenvolumen der Kammer in einem Bereich von 0,1 bis 2 ml, wobei in einer besonderes bevorzugten Ausführungsform die Kammer ein Innenvolumen von 500 Mikroliter (µl) aufweist.

Der erfindungsgemäße Fingerling erlaubt eine einfache und schnelle sowie atraumatische Versorgung von Patienten mit Fingerendgliedteilamputationen. Das gilt insbesondere auch für unkooperative Patienten, also Patienten, die sich der Behandlung insgesamt wiedersetzen oder zumindest Teile des Behandlungsregimes nicht befolgen wollen oder können. Der Fingerling ermöglicht es auf unkomplizierte Weise, einen semiokklusiven, feuchten Raum um die Wundfläche zu schaffen. Er fördert die Geweberegeneration, so dass das Ziel einer Restitutio ad integrum, d. h. eines narbenfreien Heilens, trotz des Substanzdefektes gegenüber dem Stand der Technik leichter und besser erreicht werden kann. Die erfindungsgemäß vorgesehene Kammer des Fingerlings schafft erstmals die Möglichkeit, die Gewebsregeneration wissenschaftlich, beispielsweise mikrobiologisch, molekularbiologisch, zytologisch usw., zu erforschen, ohne die Regeneration zu stören oder den Patienten in irgendeiner Weise zu beeinträchtigen. Ferner kann die Kammer die atraumatische Applikation von Mitteln zur Förderung der Regeneration von Bindegewebe und Haut ermöglichen. Der erfindungsgemäße Fingerling erlaubt ein einfaches und schnelles Abziehen vom Finger und ein ebenso einfaches und schnelles Wiederaufstecken auf den Finger.

Der Fingerling weist den Verstärkungsbereich auf, der sich von dem proximalen Ende des Fingerlings zu den Wandungen der Kammer erstreckt. Dieser Bereich kann beispielsweise streifenförmig oder stegförmig ausgebildet sein. Vorzugsweise liegt die Breite des Bereiches zwischen einem Fünftel und einem Drittel, besonders bevorzugt bei einem Viertel des Umfangs des Fingerlings. Der Ausdruck "Breite" des Bereiches bezieht sich dabei auf seine Ausdehnung in Umfangsrichtung. Beim Überziehen des Fingerlings über einen Finger liegt der Verstärkungsbereich zweckmäßigerweise auf dem Fingerrücken auf. Er kann aber auch auf der palmaren Seite des Fingers oder einem anderen Abschnitt des Fingers aufliegen. Die Lage des Verstärkungsbereichs kann so gewählt werden, dass der Verstärkungsbereich für eine Schienung, d. h. Ruhigstellung des Fingers, und darüberhinaus für einen mechanischen Schutz der Wundfläche sorgt.

Vorteilhafterweise besteht der Fingerling aus gummielastischem Material. In einer Ausführungsform besteht der Fingerling aus einem ersten gummielastischen Material und einem zweiten gummielastischen Material, wobei das zweite gummielastische Material steifer als das erste gummielastische Material ist. Aus dem zweiten gummielastischen Material können die Wandungen der Kammer ganz oder teilweise gebildet sein. Zu diesen Wandungen gehören zweckmäßigerweise nicht nur die außenliegenden Wandungen der Kammer, die gleichzeitig Teil der Außenseite des Fingerlings sind, sondern auch die innenliegende Scheidewand zwischen Kammer und Innenraum des Fingerlings. Die Scheidewand ist vorzugsweise zum Innenraum hin wie eine intakte Fingerbeere oder Fingerkuppe, also beispielsweise konkav oder annähernd konkav, geformt. Sie kann zusätzlich zur Kammer hin konkav geformt sein. Die fingerbeeren- oder fingerkuppenförmige Formung der Scheidewand ermöglicht die Regeneration des Fingerendgliedes hin zur natürlichen Form.

Zusätzlich oder alternativ zu Wandungen der Kammer kann der Verstärkungsbereich aus dem zweiten gummielastischen Material gebildet sein.

Das weichere erste gummielastische Material sorgt für einen hohen Tragekomfort des Fingerlings und ermöglicht ein abrollendes Überziehen des Fingerlings über einen Finger ähnlich wie bei einem Kondom.

Das gummielastische Material ist vorzugsweise ein gummielastisches Material auf Silikonbasis wie Silikonkautschuke oder Silikonelastomere. Im Folgenden werden gummielastische Materialien auf Silikonbasis auch als "Silikon" bezeichnet. Dabei können bekannte Silikone eingesetzt werden, wie sie beispielsweise für Chirurgie-Handschuhe üblich sind. Die Silikone sollten hautverträglich sein und auch nach wochenlangem Tragen des Fingerlings nicht an Haut oder Wundflächen haften.

Besteht der erfindungsgemäße Fingerling aus zwei gummielastischen Materialien unterschiedlicher Steifigkeit, so sind vorzugsweise beide gummielastische Materialien gummielastische Materialien auf Silikonbasis, wobei das zweite gummielastische Material auf Silikonbasis steifer als das erste gummielastische Material ist. Das erste gummielastische Material auf Silikonbasis wird im Folgenden daher auch als "weiches Silikon" bezeichnet, während das zweite gummielastische Material auf Silikonbasis als "hartes Silikon" bezeichnet wird.

Das weiche Silikon sollte eine sehr geringe Steifigkeit aufweisen und sehr dehnbar sein. Das weiche Silikon kann beispielsweise ein Silikongel sein. Durch das weiche Silikon haftet der Fingerling selbstständig an dem Finger. Dies kann auf einen Vakuumeffekt zurückgeführt werden. Um den Fingerendglieddefekt herum wird ein feuchter Raum im Wundbereich geschaffen, in dem die Geweberegeneration wie im konventionellen Folienverband erfolgen kann. Der Fingerling ist nahezu unkaputtbar und widersteht auch harten Einsatzbedingungen, insbesondere auch mechanischen Belastungen und Schmutz, wie sie bei im Freien spielenden Kindern üblicherweise auftreten.

Besteht der Fingerling nur aus einem einzigen gummielastischen Material, so kann der Verstärkungsbereich eine höhere Materialstärke als die an ihn angrenzenden Bereiche des Mantels des Fingerlings aufweisen. Besteht der Fingerling nur aus einem einzigen gummielastischen Material, so handelt es sich bei diesem Material vorzugsweise um das vorstehend als "weiches Silikon" beschriebene Material. Ein Beispiel eines geeigneten gummielastischen Materials ist ein Silikongel.

Das gummielastische Material, aus dem der Fingerling besteht, sorgt für eine optimale Passform des Fingerlings, was wiederum eine schnelle und sichere Versorgung der Wunde ermöglicht. Der Verstärkungsbereich aus dem zweiten gummielastischen Material, das im Vergleich zum ersten gummielastischen Material eine höhere Steifigkeit aufweist, liegt bei der Anwendung des Fingerlings vorzugsweise auf dem Fingerrücken auf. Damit ergibt sich gleichzeitig eine Schienung des betroffenen Fingers, wodurch dieser während der Behandlung ausreichend ruhiggestellt wird. Das gewählte gummielastische Material sollte eine ausreichende Festigkeit aufweisen, wobei die Festigkeit nicht zu hoch sein sollte, damit der Widerstand gegen Verformung gering ist und der Fingerling nicht abschnüren kann. Ferner sollte Das gewählte gummielastische Material eine ausreichend Risszähigkeit aufweisen, damit der Fingerling vor allem beim Anziehen nicht zerreißt.

Die Kammer befindet sich erfindungsgemäß am distalen Ende des Fingerlings. Damit bilden die Außenwandungen der Kammer gleichzeitig das distale Endes des Fingerlings und einen daran anschließenden Bereich des Mantels des Fingerlings. Bestehen die Außenwandungen der Kammer aus dem zweiten gummielastischen Material, beispielsweise aus hartem Silikon, so ist damit das distale Ende des Fingerlings gegen Belastungen geschützt. Damit wird insbesondere auch die Wunde, die sich bei übergestreiftem Fingerling nahe an der Scheidewand befindet, insbesondere vor mechanischen Belastungen geschützt. Vorzugsweise nimmt der Durchmesser des Fingerlings, ausgehend vom proximalen Ende, zum distalen Ende hin nicht zu.

Der Innenraum des Fingerlings und die Kammer stehen in Flüssigkeitskommunikation miteinander. Zur Herstellung einer solchen Flüssigkeitskommunikation können ein oder mehrere Einrichtungen zur Flüssigkeitskommunikation, beispielsweise ein oder mehrere Kapillaren, Kanäle, Röhren, permeable Membranen, semipermeable Membranen und Kombinationen davon ausgebildet sein. Aufgrund der Flüssigkeitskommunikation kann überschüssige Wundflüssigkeit in die Kammer ablaufen. Dabei kann freie Diffusion zur Wunde bestehen. Zweckmäßigerweise ist eine der Einrichtungen zur Flüssigkeitskommunikation axial, bezogen auf die Längsachse des Fingerlings, angeordnet.

Es kann vorgesehen sein, dass ein Flüssigkeitsdurchtritt nur vom Innenraum des Fingerlings in die Kammer, aber nicht von der Kammer in den Innenraum des Fingerlings möglich ist. Dies kann beispielsweise mit einer semipermeablen Membran erreicht werden.

Die Länge des Fingerlings kann variiert werden. Beispielsweise können lange Fingerlinge an ihrem proximalen Ende gekürzt werden, um den Fingerling an die anatomischen Gegebenheiten des zu behandelnden Fingers anzupassen. Das ist insbesondere bei Kindern und Jugendlichen zweckmäßig. Schließlich können Fingerlinge mit unterschiedlichen Durchmessern vorgesehen sein.

Der erfindungsgemäße Fingerling erlaubt ein einfaches und schnelles Schaffen einer verträglichen, geschützten semiokklusiven Kammer zur Gewebsregenration am Finger ohne zusätzliche Schienen oder Klebeverbände, welche auch unter harten Einsatzbedingungen (beispielsweise bei im Freien spielenden Kindern) verlässlichen Schutz bietet. Er bietet ferner Zugang zur Wundflüssigkeit über eine Kammer, was die Entnahme von Proben erlaubt. Diese Kammer kann auch für die Applikation von heilungsfördernden Substanzen genutzt werden.

Mittels des erfindungsgemäßen Fingerlings wird eine adäquate Versorgung auch schwerer Fingerendgliedteilamputationen schnell, atraumatisch, ohne Narkose, ohne hoch spezialisiertes Personal und ohne übermäßigen Stress für den Patienten, insbesondere auch für Kinder, erreicht. Dabei wird ein sicherer Schutz der Wunde vor äußeren Einflüssen ohne unnötig dicke Verbände, Schienen oder andere übermäßig immobilisierende Maßnahmen erzielt. Es wird ein Zugang zur Wundflüssigkeit geschaffen, ohne dabei den Patienten oder die Gewebsregeneration zu stören, so dass diagnostische (z. B. Bestimmung bakterieller Erreger) oder wissenschaftliche Daten aus der Wundflüssigkeit leichter gewonnen werden können.

In dem Teil des Mantels, der den Innenraum umschließt, können mehrere zusätzliche Öffnungen ausgebildet sein, die den Innenraum mit der Außenseite des Fingerlings verbinden. Durch diese zusätzlichen Öffnungen kann überschüssige Wundflüssigkeit, welche von der Kammer an der Spitze des Fingerlings nicht aufgenommen werden kann, kontrolliert zur Ableitung gebracht werden. Somit kann im Schaftbereich des Fingerlings diese Wundflüssigkeit kontrolliert ablaufen und von einer Kompresse oder ähnlichem aufgefangen werden. Dadurch kommt es am proximalen Ende des Fingerlings nicht zu einer Summation zweier auf die Haut einwirkender irritativer Faktoren: der Reibung durch den Rand des Fingerlings und der Feuchtigkeit durch die überschüssige Wundflüssigkeit. Dieses Prinzip der Flüssigkeitsableitung entfernt vom Rand der Silikonorthese kann auch bei anderen okkludierenden Orthesen zur Anwendung kommen.

Die Zahl der zusätzlichen Öffnungen liegt vorzugsweise zwischen zwei und zehn. Die Öffnungen haben vorzugsweise einen Durchmesser von etwa 0,5 bis 2 mm, besonders bevorzugt etwa 1 mm. Die zusätzlichen Öffnungen können auch als kapilläre Verbindungen zwischen dem Innenraum und der Außenseite des Fingerlings ausgebildet sein.

Nach Maßgabe der Erfindung ist ferner ein Kit vorgesehen, das erfindungsgemäße Fingerlinge umfasst, wobei die Fingerlinge unterschiedliche Durchmesser aufweisen. Zusätzlich können die Fingerlinge auch unterschiedliche Längen und/oder Kammern unterschiedlicher Größe aufweisen. Vorzugsweise umfasst das Kit Fingerlinge mit Durchmessern in fünf bis zwölf, bevorzugt neun unterschiedlichen Größen. Mit neun verschiedenen Größen lassen sich fast alle Fingergrößen und Verletzungen von Kindern und Jugendlichen zwischen 0 und 15 Jahren abdecken.

Das Kit kann ferner ein Messinstrument zur Bestimmung von Fingerdurchmessern umfassen. Dabei kann es sich um ein Set an Messringen handeln, mit dem sich am korrespondierenden Finger der gegenseitigen Hand schnell und einfach der Fingerdurchmesser ermitteln lässt. Auf Basis des so gewonnenen Messergebnisses kann dann der benötigte Durchmesser des Fingerlings bestimmt und ein Fingerling ausgewählt werden, dessen Durchmesser dem gemessenen Fingerdurchmesser entspricht.

Der Fingerling kann insbesondere zur Behandlung von distalen Fingerverletzungen, insbesondere von Fingerendgliedteilamputationen oder andern Fingerendglieddefekten verwendet werden. Er kann aber auch zur Behandlung anderer Verletzungen mit Substanzdefekt eingesetzt werden, beispielsweise für distale Zehenverletzungen. Eine optional vorgesehene teilweise Verstärkung der Wand des Fingerlings kann eine ausreichende Ruhigstellung des verletzten Gliedes bewirken. Eine optional vorgesehene Verstärkung des distalen Endes und der Wandung(en) der Kammer kann die Wundfläche optimal vor Umwelteinflüssen schützen.

Die Erfindung wird nachstehend anhand eines Ausführungsbeispiels, das die Erfindung nicht einschränken soll, unter Bezugnahme auf die Zeichnungen näher erläutert.

### Dabei zeigen

- Fig. 1: eine Draufsicht einer ersten Ausführungsform eines erfindungsgemäßen Fingerlings;
- Fig. 2: eine Schnittdarstellung der in Fig. 1 gezeigten ersten Ausführungsform des erfindungsgemäßen Fingerlings entlang Schnittlinie A-A, wobei das Verstärkungselement in Fig. 2 zur Vereinfachung der Darstellung nicht gezeigt ist;
- Fig. 3: eine Draufsicht einer zweiten Ausführungsform eines erfindungsgemäßen Fingerlings;
- Fig. 4: eine Draufsicht einer dritten Ausführungsform eines erfindungsgemäßen Fingerlings; und
- Fig. 5: eine Draufsicht einer vierten Ausführungsform eines erfindungsgemäßen Fingerlings.

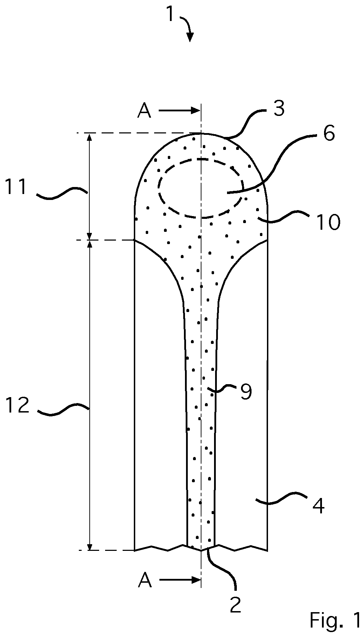

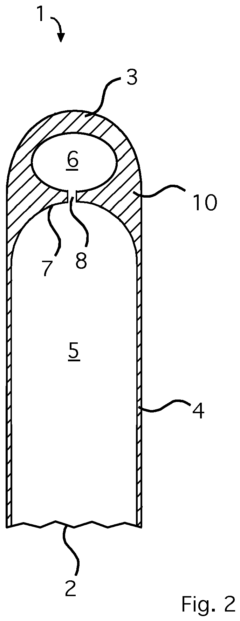

Die in den Figuren 1 und 2 gezeigte erste Ausführungsform des Fingerlings 1 weist ein proximales offenes Ende 2, ein distales geschlossenes Ende 3 und einen Mantel 4 auf, der sich von dem proximalen Ende 2 zum distalen Ende 3 erstreckt. Der Mantel 4 umschließt einen Innenraum 5 und eine Kammer 6, die durch eine Scheidewand 7 von dem Innenraum 5 getrennt ist. In der Scheidewand 7 ist eine Kapillare 8 ausgebildet, die den Innenraum 5 mit der Kammer 6 verbindet und die Flüssigkeitskommunikation zwischen Innenraum 5 und Kammer 6 ermöglicht. Der Hohlraum der Kammer 6 hat eine elliptische Form. Die Scheidewand 7 ist zum Innenraum 5 hin fingerbeerenförmig geformt. Die Kammer 6 befindet sich am distalen Ende 3 des Fingerlings 1. Die Außenwandung 10 der Kammer 6 ist gleichzeitig Teil der Außenseite des Fingerlings 1. Wird der Fingerling 1 über einen Finger gezogen, gelangt der Finger in den Innenraum 5 des Fingerlings 1.

Wie in Fig. 1 zu erkennen ist, weist der Mantel 4 einen streifenförmigen Verstärkungsbereich 9 auf, der sich vom proximalen Ende 2 des Fingerlings 1 bis zu der Kammer 6 erstreckt. Wird der Fingerling 1 über einen Finger gezogen, so soll der Verstärkungsbereich 9 dorsal auf dem Finger aufliegen.

Die Kammer 6, die von der Außenwandung 10 und der Scheidewand 7 unter Ausbildung eines Hohlraums gebildet ist, sowie der Verstärkungsbereich 9 des Mantels 4 bestehen aus hartem Silikon. Die übrigen Teile des Fingerlings, also der Mantel 4 mit Ausnahme des Verstärkungsbereiches 9, bestehen aus weichem Silikon. Sowohl das harte Silikon als auch das weiche Silikon sind gummielastische Materialien. Es ist in den Figuren 1 und 2 zu erkennen, dass am distalen Ende des Fingerlings 1 ein Abschnitt 11 aus hartem Silikon ausgebildet ist, in dem sich die Kammer 6 befindet. An diesen Abschnitt 11 schließt sich ein Abschnitt 12 aus - abgesehen vom Verstärkungselement 9 - weichem Silikon an, der sich bis zum proximalen Ende des Fingerlings 1 erstreckt. In diesem Abschnitt 12 ist das streifenförmige Verstärkungselement 9 aus hartem Silikon ausgebildet.

Auf die Außenseite des Mantels 4 kann eine Größenangabe, beispielsweise Nummern, aufgebracht sein, die sich auf den Umfang im Grundgliedbereich der zur Herstellung genutzten Gipsmodelle beziehen, welche anhand umfangreicher Messungen an Kinderhänden entstanden sind.

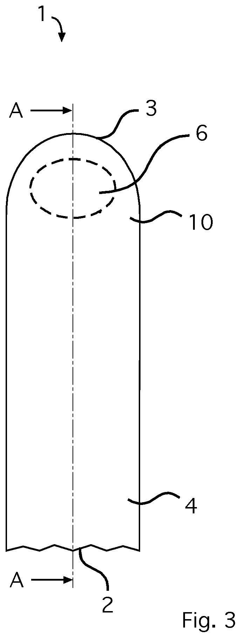

Die in Fig. 3 gezeigte zweite Ausführungsform eines erfindungsgemäßen Fingerlings entspricht der ersten Ausführungsform, außer dass der Fingerling 1 nur aus einem einzigen gummielastischen Material besteht, nämlich dem in der ersten Ausführungsform verwendeten weichen Silikon, und kein Verstärkungsbereich ausgebildet ist.

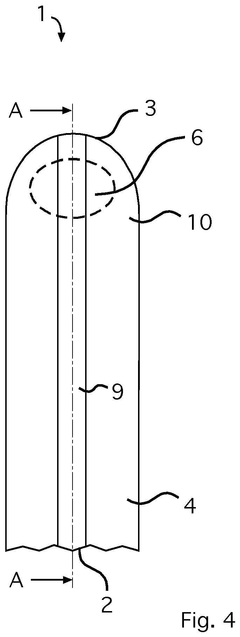

Die in Fig. 4 gezeigte dritte Ausführungsform eines erfindungsgemäßen Fingerlings entspricht der ersten Ausführungsform, außer dass der Fingerling 1 nur aus einem einzigen gummielastischen Material besteht, nämlich dem in der ersten Ausführungsform verwendeten weichen Silikon. Der Verstärkungsbereich 9 weist eine höhere Materialstärke auf als die an seine Längsränder angrenzenden Bereiche des Mantels des Fingerlings 1.

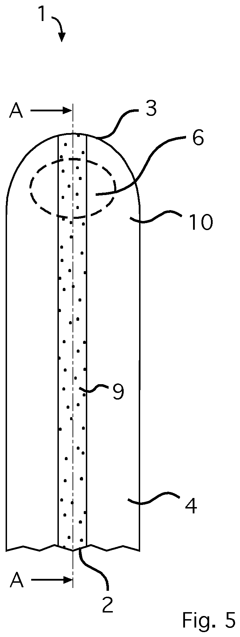

Die in Fig. 5 gezeigte vierte Ausführungsform eines erfindungsgemäßen Fingerlings entspricht der ersten Ausführungsform, außer dass nur der Verstärkungsbereich 9 des Fingerlings 1 aus hartem Silikon besteht, nicht aber die Wandungen der Kammer 6.

### Bezugszeichenliste

- 1: Fingerling
- 2: proximales Ende
- 3: distales Ende
- 4: Mantel
- 5: Innenraum
- 6: Kammer
- 7: Scheidewand
- 8: Kapillare
- 9: Verstärkungsbereich
- 10: Außenwandung
- 11: Abschnitt aus hartem Silikon
- 12: Abschnitt aus weichem Silikon

## Patentansprüche

1. Fingerling, insbesondere für die Behandlung von Fingerendglieddefekten, mit einem geschlossenen distalen Ende (3), einem offenen proximalen Ende (2) und einem Innenraum (5), wobei in dem Fingerling (1) an dessen distalem Ende (3) eine Kammer (6) ausgebildet ist, wobei die Kammer (6) mit dem Innenraum (5) des Fingerlings (1) in Flüssigkeitskommunikation steht und der Fingerling einen Verstärkungsbereich (9) aufweist, der sich von dem proximalen Ende (2) des Fingerlings (1) zu den Wandungen (7, 10) der Kammer erstreckt.

2. Fingerling nach Anspruch 1, **dadurch gekennzeichnet, dass** er aus gummielastischem Material besteht.

3. Fingerling nach Anspruch 1 oder Anspruch 2, **dadurch gekennzeichnet, dass** er aus einem ersten gummielastischen Material und einem zweiten gummielastischen Material besteht, wobei das zweite gummielastische Material steifer als das erste gummielastische Material ist und wobei die Wandungen (7, 10) der Kammer (6) aus dem zweiten gummielastischen Material gebildet sind.

4. Fingerling nach Anspruch 3, **dadurch gekennzeichnet, dass** der Verstärkungsbereich aus dem zweiten gummielastischen Material gebildet ist.

5. Fingerling nach einem der Ansprüche 1 bis 4, **dadurch gekennzeichnet, dass** der Bereich (9) streifen- oder stegförmig ausgebildet ist.

6. Fingerling nach einem der Ansprüche 3 bis 5, **dadurch gekennzeichnet, dass** eine Kapillare (8) axial in dem Fingerling (1) verläuft.

7. Fingerling nach einem der vorstehenden Ansprüche, **dadurch gekennzeichnet, dass** der von den Wandungen (7, 10) umschlossene Hohlraum im Wesentlichen kugelförmig oder ellipsenförmig ist.

8. Fingerling nach einem der vorstehenden Ansprüche, **dadurch gekennzeichnet, dass** er aus gummielastischem Material auf Silikonbasis besteht.

9. Kit, umfassend Fingerlinge nach einem der Ansprüche 1 bis 8, **dadurch gekennzeichnet, dass** es Fingerlinge (1) mit unterschiedlichen Durchmessern umfasst.

10. Kit nach Anspruch 9, **dadurch gekennzeichnet, dass** es ferner zumindest ein Messinstrument zur Bestimmung von Fingerdurchmessern umfasst.

## Claims

1. A fingerstall, in particular for the treatment of defects of the distal phalanx of the finger with a closed distal end (3), an open proximal end (2), and an interior (5), wherein a chamber (6) is formed in the fingerstall (1) at the distal end (3) thereof, wherein the chamber (6) is in liquid communication with the interior (5) of the fingerstall (1) and the fingerstall has a reinforced region (9) extending from the proximal end (2) of the fingerstall (1) to the walls (7, 10) of the chamber.

2. The fingerstall according to claim 1, **characterized in that** it consists of a rubber-elastic material.

3. The fingerstall according to claim 1 or claim 2, **characterized in that** it consists of a first rubber-elastic material and a second rubber-elastic material, wherein the second rubber-elastic material is more rigid than the first rubber-elastic material and wherein the walls (7, 10) of the chamber (6) are formed of the second rubber-elastic material.

4. The fingerstall according to claim 3, **characterized in that** the reinforced region is formed of the second rubber-elastic material.

5. The fingerstall according to any one of claims 1 to 4, **characterized in that** the region (9) is in form of stripes or ribs.

6. The fingerstall according to any one of claims 3 to 5, **characterized in that** a capillary (8) extends axially in the fingerstall (1).

7. The fingerstall according to any one of the preceding claims, **characterized in that** the cavity formed by the walls (7, 10) is substantially spherical or elliptical.

8. The fingerstall according to any one of the preceding claims, **characterized in that** it consists of a silicone-based rubber-elastic material.

9. A kit comprising fingerstalls according to any one of claims 1 to 8, **characterized in that** it comprises fingerstalls (1) of different diameters.

10. The kit according to claim 9, **characterized in that** it further comprises at least one measuring instrument for determining finger diameters.

## Revendications

1. Doigtier, notamment pour le traitement de défauts de bout de doigts, avec une extrémité distale fermée (3), une extrémité proximale ouverte (2) et un intérieur (5) où dans le doigtier (1) une chambre (6) est formée à cette extrémité distale (3), tandis que la chambre (6) se trouve en communication liquide avec l'intérieur (5) du doigtier (1) et le doigtier présente une zone de renfort (9) qui s'étend de l'extrémité proximale (2) du doigtier (1) aux parois (7, 10) de la chambre.

2. Doigtier selon revendication 1, de ce fait **caractérisé en ce qu'**il se compose d'un matériau en caoutchouc élastique.

3. Doigtier selon revendication 1 ou revendication 2, de ce fait **caractérisé en ce qu'**il se compose d'un premier matériau en caoutchouc élastique et d'un deuxième matériau en caoutchouc élastique, tandis que le deuxième matériau en caoutchouc élastique est plus raide que le premier matériau en caoutchouc élastique et tandis que les parois (7, 10) de la chambre (6) sont formées à partir du deuxième matériau en caoutchouc élastique.

4. Doigtier selon revendication 3, de ce fait **caractérisé en ce que** la zone de renfort est formée à partir d'un deuxième matériau en caoutchouc élastique.

5. Doigtier selon revendications 1 à 4, de ce fait **caractérisé en ce que** la zone (9) est formée de rayures ou de traverses.

6. Doigtier selon revendications 3 à 5, de ce fait **caractérisé en ce qu'**un tube capillaire (8) s'étend dans l'axe du doigtier (1).

7. Doigtier selon les revendications susmentionnées, de ce fait **caractérisé en ce que** le creux entouré par les parois (7, 10) est essentiellement en forme de boules ou d'ellipses.

8. Doigtier selon les revendications susmentionnées, de ce fait **caractérisé en ce qu'**il se compose d'un matériau en caoutchouc élastique à base de silicone.

9. Kit regroupant les doigtiers selon l'une des revendications 1 à 8, de ce fait **caractérisé en ce qu'**il regroupe les doigtiers (1) avec diamètres différents.

10. Kit selon revendication 9, de ce fait **caractérisé en ce qu'**il regroupe en outre au moins un instrument de mesure pour la détermination des diamètres de doigt.
